(19) **Europäisches Patentamt / European Patent Office / Office européen des brevets**

(11) **EP 3 919 664 A1**

(12) **EUROPEAN PATENT APPLICATION**
published in accordance with Art. 153(4) EPC

(43) Date of publication:
**08.12.2021 Bulletin 2021/49**

(21) Application number: **20749383.4**

(22) Date of filing: **30.01.2020**

(51) Int Cl.:
*D04B 1/16* (2006.01)      *D04B 21/16* (2006.01)
*A61F 2/01* (2006.01)      *A61F 2/04* (2013.01)
*A61L 33/10* (2006.01)      *A61B 17/12* (2006.01)
*D06M 15/03* (2006.01)

(86) International application number:
**PCT/JP2020/003358**

(87) International publication number:
**WO 2020/158847 (06.08.2020 Gazette 2020/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.01.2019 JP 2019013782**

(71) Applicant: **Toray Industries, Inc.**
**Tokyo, 103-8666 (JP)**

(72) Inventors:
• **KAKIYAMA, So**
  **Otsu-shi, Shiga 520-8558 (JP)**
• **YAMADA, Satoshi**
  **Otsu-shi, Shiga 520-2141 (JP)**
• **TANAKA, Nobuaki**
  **Otsu-shi, Shiga 520-2141 (JP)**
• **TANAHASHI, Kazuhiro**
  **Otsu-shi, Shiga 520-8558 (JP)**

(74) Representative: **Kador & Partner PartG mbB**
**Corneliusstraße 15**
**80469 München (DE)**

(54) **MEDICAL BASE MATERIAL FOR INDWELLING CARDIOVASCULAR DEVICE**

(57) The present invention provides a medical base material for an indwelling cardiovascular device, in which a unique knitted structure made of multifilaments containing ultra-fine fibers can maintain the antithrombotic property through the early endothelialization and has improved storage property in a sheath catheter and mechanical strength. Provided is a medical base material for an indwelling cardiovascular device, comprising a knitted fabric made of multifilaments containing 30 wt% or more of ultra-fine fibers having a single thread diameter of 1 $\mu$m to 10 $\mu$m, and heparin, a heparin derivative, or a pharmaceutically acceptable salt thereof, which is chemically bound to the surface of the ultra-fine fibers, wherein: the knitted fabric has a basis weight of 5 mg/cm$^2$ to 20 mg/cm$^2$, a thickness of 200 $\mu$m or less, and a water permeability of 1000 mL/min/cm$^2$ to 10,000 mL/min/cm$^2$ at a pressure of 120 mmHg.

Fig. 1

EP 3 919 664 A1

**Description**

TECHNICAL FIELD

The present invention relates to a medical base material for an indwelling cardiovascular device.

BACKGROUND ART

There are numerous devices which suppress thrombus formation preferably by being coated with a tissue or neointima derived from a living organism, including indwelling cardiovascular devices such as artificial blood vessels, stents, stent grafts, artificial valves and left atrial appendage occlusion devices. In recent years, in order to improve the QOL of patients, there is a growing demand for a medical device that can be used for minimally invasive endovascular treatments.

An indwelling cardiovascular device which is minimally invasive as described above is delivered to the affected area while being stored in a catheter for transport called a delivery catheter. Therefore, the indwelling cardiovascular device is formed thin so that it can be stored in the delivery catheter. On the other hand, when the medical device is formed too thin, the mechanical strength is reduced and the area where cells adhere is reduced too, resulting in the possibility of incomplete coating by a tissue or neointima derived from a living organism upon the thrombus formation. In order to prevent this, a medical device which is constituted by a mesh base material made of fibers and is thus capable of being coated by a tissue derived from a living organism in a three-dimensional structure has been devised (Non-Patent Document 1).

In order to coat the surface of the medical device with vascular endothelial cells at an early stage after the medical device is indwelled in a blood vessel, a cell scaffold material with improved cell adhesion and proliferation properties by optimizing the diameter of ultra-fine fibers in the multifilament and aligning the orientation thereof (Patent Document 1), and an artificial blood vessel in which an ultra-fine fiber having a fiber fineness of 0.5 dtex or less is used and is bound with an antithrombotic material (Patent Document 2) have been reported.

It has also been reported that an antithrombotic material in which an anticoagulant heparin is supported on the surface of polyester fibers to impart antithrombotic property is applied to an indwelling device for an endovascular treatment such as a stent graft or an artificial valve (Patent Document 3).

On the other hand, in addition to the medical use, knitted fabrics in which a multifilament containing ultra-fine fibers is used are known for general industrial use (Patent Documents 4 to 8).

PRIOR ART DOCUMENTS

PATENT DOCUMENTS

> Patent Document 1: WO 2016/068279
> Patent Document 2: WO 2015/080177
> Patent Document 3: WO 2014/168198
> Patent Document 4: JP 2001-254250 A
> Patent Document 5: JP 2000-265343 A
> Patent Document 6: WO 2013/065688
> Patent Document 7: JP 2017-206790 A
> Patent Document 8: JP 2018-172813 A

NON-PATENT DOCUMENTS

Non-Patent Document 1: Marek Grygier et al., Advances in Interventional Cardiology, 2017, Vol. 13, No. 42, pp. 62-66

SUMMARY OF THE INVENTION

PROBLEMS TO BE SOLVED BY THE INVENTION

The base fabric used in the device described in Non-Patent Document 1 is not constituted by a multifilament containing ultra-fine fibers. In this case, since the surface of this knitted fabric is not subjected to an antithrombotic treatment, the knitted fabric becomes the starting point of the thrombus formation before the knitted fabric is coated by neointima.

The scaffolding material of Patent Document 1 describes a woven fabric in which cell adhesion and proliferation properties are improved by controlling the fiber diameter and fiber orientation. However, Patent Document 1 does not describe the control of the knitting density. In the case of a knitted fabric having a high knitting density, the knitted fabric cannot be stored in a catheter when used as a member of an indwelling cardiovascular device, and thus cannot be delivered to the affected area.

Patent Document 2 describes a tubular woven artificial blood vessel in which the blood permeability through the base fabric is suppressed low by the ultra-high density weaving of multifilaments and the surface of the woven fabric is treated with heparin. However, Patent Document 2 is an invention related to an artificial blood vessel to be transplanted by surgery, and there is no aspect of storage property in a sheath catheter. Further, since the wall of the artificial blood vessel is hard due to the high-density weaving and has a thick structure, the artificial blood vessel is difficult to be stored in the sheath catheter.

Patent Document 3 discloses a method of immobilizing heparin firmly on a fiber surface by ionic bond via a cationic polymer, and thrombus formation on the fiber surface can be thus inhibited. However, since heparin is generally known to inhibit the adhesion and proliferation of vascular endothelial cells, when these techniques are simply used for indwelling cardiovascular devices, the coating by vascular endothelial cells can be delayed.

Patent Documents 4 and 5 describe knitted fabrics containing ultra-fine fibers, but multifilaments containing ultra-fine fibers are not heparinized. Further, since the thickness of the knitted fabrics is as thick as 400 $\mu$m or more, the knitted fabrics cannot be stored in a general sheath catheter.

Patent Documents 6 and 7 describe knitted fabrics having approximately the same number of stitches, but the filaments are not heparinized and ultra-fine fibers are not used, resulting in inferior affinity with cells and living tissues. Further, since the thickness of the knitted fabrics is as thick as 400 $\mu$m or more, the knitted fabrics cannot be stored in a general sheath catheter.

Patent Document 8 describes a knitted fabric for sportswear using ultra-fine fibers and having an extremely high knitting density, but the multifilaments containing the ultra-fine fibers are not heparinized. Furthermore, because of the high knitting density, the fiber bundles made of adjacent multifilaments are pressed against each other and are spread in the thickness direction, resulting in a thicker knitted fabric. Thus, the knitted fabric cannot be stored in a general sheath catheter.

As described above, in the prior art, the storage property in a general sheath catheter and the mechanical strength of the base fabric were insufficient. Furthermore, the antithrombotic property through early endothelialization required for a medical base material for an indwelling cardiovascular device could not be achieved, either.

Therefore, the present invention provides a medical base material for an indwelling cardiovascular device, which can maintain the antithrombotic property through the early endothelialization and has good storage property in a general sheath catheter and good mechanical strength.

SOLUTIONS TO THE PROBLEMS

After intensive research to solve the above problems, the inventions (1) to (5) have been achieved.

(1) A medical base material for an indwelling cardiovascular device, comprising a knitted fabric made of multifilaments containing 30 wt% or more of ultra-fine fibers having a single thread diameter of 1 $\mu$m to 10 $\mu$m, and heparin, a heparin derivative, or a pharmaceutically acceptable salt thereof, which is chemically bound to the surface of the ultra-fine fibers, wherein: the knitted fabric has a basis weight of 5 mg/cm$^2$ to 20 mg/cm$^2$, a thickness of 200 $\mu$m or less, and a water permeability of 1000 mL/min/cm$^2$ to 10,000 mL/min/cm$^2$ at a pressure of 120 mmHg.

(2) The medical base material according to (1), wherein the knitted fabric has 50 to 130 courses per 2.54 cm and 50 to 130 wales per 2.54 cm.

(3) The medical base material according to (1) or (2), wherein the average diameter of maximum circles inscribed in gaps between stitches of the knitted fabric is 80 $\mu$m or less.

(4) The medical base material according to any one of (1) to (3), wherein the knitted fabric has a tensile elongation at break of 50% or more.

(5) The medical base material according to any one of (1) to (4), wherein the knitted fabric has a tensile elastic modulus of 1 MPa to 100 MPa.

EFFECTS OF THE INVENTION

According to the present invention, by controlling the knitting density and the mesh opening of a knitted structure made of multifilaments containing ultra-fine fibers and immobilizing heparin or the like on the surface of the ultra-fine fibers, a medical base material for an indwelling cardiovascular device having good storage property in a sheath catheter and good mechanical strength can be provided.

BRIEF DESCRIPTION OF THE DRAWINGS

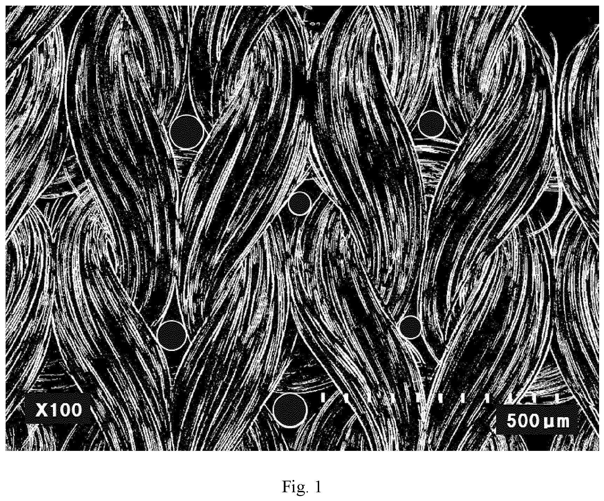

Figure 1 is an image of the medical base material for an indwelling cardiovascular device of the present invention, photographed with a scanning electron microscope at a magnification of 100 times from the direction perpendicular to the stitches of the knitted fabric.

EMBODIMENTS OF THE INVENTION

The medical base material for an indwelling cardiovascular device of the present invention can maintain the antithrombotic property through the early endothelialization and has storage property in a general sheath catheter and mechanical strength.

The medical base material as described above is used as a member of an indwelling cardiovascular device, and can be suitably used as a medical device for cardiovascular implants in particular. The indwelling cardiovascular device is a device that is stored in a sheath catheter, delivered to the affected area by a catheter inserted into a blood vessel, and indwelled in the affected area of the heart or blood vessel to exhibit effect in treating the heart or blood vessel. Examples thereof include stents, stent grafts, left atrial appendage occlusion devices, vascular embolization materials, artificial valves, coils and filters for thrombus capture.

The medical base material as described above uses a knitted fabric made of multifilaments containing ultra-fine fibers. A multifilament refers to a fiber bundle formed by a plurality of ultra-fine fibers bundled, and the single thread diameter of the ultra-fine fibers is 1 $\mu$m to 10 $\mu$m. When the single thread diameter of the ultra-fine fibers is larger than 10 $\mu$m, the cell adhesion is decreased, the thickness of the base material is increased, and the storage property in the sheath catheter is also lowered. When the single thread diameter of the ultra-fine fibers is smaller than 1 $\mu$m, the cell adhesion is reduced.

The multifilament containing the ultra-fine fibers is not limited to one type, and a plurality of types of multifilaments having different single yarn fineness and total fineness can be combined. As the multifilament, a multifilament of so-called direct spinning type may be used as it is, but a multifilament of splitting type may also be used. As the splitting type, fibers that can be made ultra-fine by chemical or physical means can be used. Further, after a knitted fabric is formed, by making some fibers in the knitted fabric ultra-fine, it is possible to obtain a multifilament containing ultra-fine fibers. As a method of making fibers ultra-fine by chemical or physical means, as described in US Patent No. 3,531,368 and US Patent No. 3,350,488, fibrils or ultra-fine fibers can be obtained by, for example, removing or detaching one component of the multi-component fibers.

As the multi-component fiber described above, a sea-island composite fiber is known. By removing the sea component, the island component constitutes the above knitted fabric as part of the ultra-fine fiber multifilament. The number of islands of the sea-island composite fiber, that is, the number of single yarns of the ultra-fine fibers contained in the sea-island composite fiber is not particularly limited.

The knitted fabric as described above needs to contain ultra-fine fibers, but may contain thick fibers other than the ultra-fine fibers. In particular, the knitted fabric may use thick fibers having a diameter of 11 $\mu$m or more in order to exhibit mechanical properties. When the ratio of the ultra-fine fibers is too small, the cell adhesion property is lowered and it becomes difficult for the cells to invade the medical base material and form new tissues. Therefore, the weight ratio of the ultra-fine fibers in the multifilament is preferably 30 wt% or more, and more preferably 50 wt% or more. The weight ratio of the ultra-fine fibers in the multifilament can be measured as follows: a multifilament constituting the loops of a knitted fabric is unraveled from the knitted fabric and measured for the total weight per unit length; the weight is measured after thick fibers of 11 $\mu$m or more are removed; and then the percentage to the total weight can be calculated.

The raw materials of the above-mentioned ultra-fine fibers and thick fibers are not limited, but are preferably a polymer selected from the group consisting of polyester, polypropylene, nylon, acrylic, polyamide and polystyrene, particularly preferably polyester because of the proven track record in medical applications. Among polyesters, polyethylene terephthalate or polybutylene terephthalate is preferred.

The thickness of the medical base material needs to be 200 $\mu$m or less, and is preferably 180 $\mu$m or less.

The fiber density of the knitted fabric has a great influence on the mechanical strength, the water permeability at a pressure of 120 mmHg, and the cell adhesion property of the above-mentioned medical base material. The fiber density of the knitted fabric herein is determined by the basis weight and the number of stitches.

The basis weight of the knitted fabric indicates the weight of the knitted fabric per unit area of the spread surface of the knitted fabric. The basis weight of the knitted fabric needs to be 5 mg/cm$^2$ to 20 mg/cm$^2$, and the upper limit thereof is particularly preferably 18 mg/cm$^2$ or less.

The number of stitches in the knitted fabric is expressed by the number of loops in the weft direction (number of courses) and the number of loops in the warp direction (number of wales) of the knitted fabric. The number of courses per 2.54 cm is preferably 50 to 130, and the number of wales per 2.54 cm is preferably 50 to 130. Furthermore, the number of courses per 2.54 cm is more preferably 75 to 110, and the number of wales per 2.54 cm is more preferably 75 to 110. The number of stitches within the above range results in an appropriate thickness. Therefore, the storage property in a general sheath catheter increases, and the appropriate mechanical strength does not prevent the coating by neointimal tissues and improves the cell adhesion area.

The mesh opening of the knitted fabric is expressed as the diameter of a gap formed in the stitches of the knitted fabric made of multifilaments. However, since the shape of the gap varies depending on the knitting method, there is no standard measurement method. Therefore, for convenience, the mesh opening of the knitted fabric was defined based on the maximum circles inscribed in the gaps of the stitches detected as a portion without any thread in the image taken from the direction perpendicular to the stitches of the knitted fabric. Specifically, the gaps between the stitches in the obtained image were randomly selected, and the gaps between the stitches were approximated by circles to obtain the maximum circles inscribed in the gaps between the stitches. The diameters of the maximum circles inscribed in the gaps were measured and used as the mesh opening of the knitted fabric.

The gaps between the stitches were measured at 10 points, and the average value was used as the average value of mesh opening. That is, the average value of mesh opening of the knitted fabric is indicated by the average value of the diameters of the maximum circles inscribed in the gaps of the knitted fabric.

The average value of mesh opening of the knitted fabric is preferably 80 $\mu$m or less, more preferably 20 $\mu$m to 60 $\mu$m. Within this range, cells can easily invade the gaps between the stitches, and the coating by neointima can be obtained more stably. When the average value of mesh opening is larger than 80 $\mu$m, cells are less likely to interact with each other and endothelialization is delayed.

The type of the knitted fabric as described above is not particularly limited, and the flat knitted fabric (T cloth), 1x1 T-cloth, moss knitting, rib knitting, double-sided knitting, purl knitting, blister knitting, single denbigh knitting, single cord knitting, single atlas knitting, tricot knitting, half tricot knitting, double denbigh knitting, satin knitting and the like are used. Tricot knitting or half tricot knitting which allows for a high fiber density structure is preferably used.

Medical devices for an endovascular treatment, which use the above medical base material, include devices that are indwelled and used in the ventricles, atriums, or arteries that are affected by the heartbeat. Thus, the medical base material needs to have mechanical strength which is not affected by the heartbeat. The mechanical strength of the medical base material is characterized by two independent indicators of tensile elongation at break and tensile elastic modulus of the medical base material.

The tensile elongation at break of the medical base material herein is preferably 1% to 20%, and particularly preferably 5% to 15%. When the tensile elongation at break is 20% or less, the disturbed blood flow due to the expansion and contraction of the medical base material can be prevented, and also, the detachment of the cells adhering to the surface of the medical base material can be prevented thanks to the multifilaments sliding on each other. Further, when the tensile elongation at break is 1% or more, the distortion of the medical base material due to the heartbeat can be prevented.

The tensile elastic modulus of the medical base material is preferably 1 MPa to 100 MPa, and particularly preferably 10 MPa to 80 MPa. When the tensile elastic modulus is 100 MPa or less, the medical base material can stably follow the movement of the heart and blood vessels, and thus, the damage to surrounding tissues can be prevented. Further, when the tensile elongation at break is 1 MPa or more, the distortion due to the heartbeat can be prevented.

The water permeability of the medical base material at a pressure of 120 mmHg is preferably 1000 mL/min/cm$^2$ to 10,000 mL/min/cm$^2$, and particularly preferably 2000 mL/min/cm$^2$ to 9000 mL/min/cm$^2$. Within this range, cells can easily invade the gaps of the multifilament while the mechanical strength is maintained, and the coating by neointima can be obtained more stably. When the water permeability at a pressure of 120 mmHg is lower than 1000 mL/min/cm$^2$, cell invasion is hindered and endothelialization is delayed, and when the water permeability at a pressure of 120 mmHg is greater than 10,000 mL/min/cm$^2$, the mechanical strength of the knitted fabric itself is decreased.

The above medical base material is chemically bound with heparin, a heparin derivative, or a pharmaceutically acceptable salt thereof on the surface of the ultra-fine fibers. As the heparin or the heparin derivative, low molecular weight heparin is particularly preferably used. The low molecular weight heparin herein refers to a fractionated heparin derivative having a weight average molecular weight of 2000 to 5000 obtained by heparin digestion by an enzyme or a chemical treatment.

When the above low molecular weight heparin is used, reviparin, enoxaparin, parnaparin, certoparin, dalteparin and tinzaparin, and pharmaceutically acceptable salts thereof can be preferably used because they are used clinically.

When heparin, a heparin derivative or a pharmaceutically acceptable salt thereof is chemically bound to the surface of the ultra-fine fibers constituting the knitted fabric, the abundance of the heparin or the heparin derivative on the surface of the knitted fabric of the medical base material can be quantified by X-ray photoelectron spectroscopy (XPS). For the abundance of the heparin or the heparin derivative on the surface of the knitted fabric of the medical base material, the abundance ratio of sulfur atoms on the surface of the knitted fabric of the medical base material can be used as an index. When the surface of the knitted fabric of the medical base material is measured by X-ray electron spectroscopy (XPS), the abundance ratio of sulfur atoms with respect to the abundance of all the atoms is preferably 3.0 at% to 6.0 at%.

The method of chemically binding heparin, a heparin derivative or a pharmaceutically acceptable salt thereof to the surface of the ultra-fine fibers constituting the knitted fabric is not particularly limited. Known methods can be used, such as a method of immobilization by covalent bonding with a functional group introduced onto the surface of a base material (JP 4152075 B, JP 3497612 B or JP H10-513074 A), and a method of immobilization by ionic bonding with a positively charged cationic compound introduced onto the surface of a base material (JP S60-041947 B, JP S60-047287 B, JP 4273965 B or JP H10-151192 A). A surface support of sustained-release in which heparin is bound by ionic bond is preferred because, until the surface is coated by the neointima, the antithrombotic property is exhibited while the coating by the neointima is not inhibited. The method described in WO 2015/080177 is particularly preferably used.

The antithrombotic property of the chemically bound heparin, heparin derivative or pharmaceutically acceptable salt thereof can be confirmed by quantifying the thrombin-antithrombin complex (hereinafter, "TAT"). Better antithrombotic property can suppress more the formation of thrombus on the surface of the medical base material, and the lower TAT value is more preferred. For clinical use as the indwelling cardiovascular device of the present invention, the TAT needs to be 50 ng/mL/cm$^2$ or less.

EXAMPLES

The present invention will be described in detail with reference to Examples and Comparative Examples below, but the present invention is not limited thereto.

(Example 1)

Using a weft knitting machine, a sheet-like knitted fabric was prepared from a multifilament yarn made of 9 filaments of sea-island composite fibers (70 islands/filament) with a total fineness of 66 dtex, and a flat knitted fabric having 70 wales per 2.54 cm and 70 courses per 2.54 cm after the removal treatment of the sea component was produced. The sea-island composite fiber is constituted by polyethylene terephthalate in the island component and polyethylene terephthalate copolymerized with 5-sodium sulfoisophthalate in the sea component.

Then, in order to perform the removal treatment of the sea component, the flat knitted fabric was subjected to the following (c-1) acid treatment step and (c-2) alkali treatment step. Thus, a knitted fabric 1, which was a flat knitted fabric constituted by multifilaments containing ultra-fine fibers was obtained.

(C-1) Acid treatment step

Maleic acid was used as the acid. In the acid treatment, the flat knitted fabric was immersed in an aqueous solution of 0.2 wt% maleic acid, heated to 130°C, and then heated for 30 minutes.

(C-2) Alkali treatment step

Sodium hydroxide was used as the alkali. In the alkali treatment, the flat knitted fabric was immersed in an aqueous solution of 1 wt% sodium hydroxide, heated to 80°C, and then heated for 90 minutes.

The obtained knitted fabric 1 was subjected to an antithrombotic treatment. The knitted fabric 1 was immersed in an aqueous solution containing 0.6 mol/L sulfuric acid and 3.0 wt% potassium permanganate (manufactured by Wako Pure Chemical Industries, Ltd.) and reacted at 60°C for 3 hours to hydrolyze and oxidize the surface of the knitted fabric 1 (hydrolysis and oxidization step). After the reaction, the aqueous solution was removed, and the resulting knitted fabric was washed 3 times with an aqueous solution containing 6 mol/L hydrochloric acid and once with distilled water.

The flat knitted fabric was immersed in an aqueous solution containing 2.0 wt% 4(-4,6-dimethoxy-1,3,5-triazin-2-yl)-4-methylmorpholinium chloride n-hydrate (hereinafter, "DMT-MM") (manufactured by Wako Pure Chemical Industries, Ltd.) and 5.0 wt% polyethyleneimine (LUPASOL (registered trademark) P; manufactured by BASF; weight average molecular weight: 750,000) and reacted at 50°C for 2 hours to covalently bind the polyethyleneimine to the surface of the knitted fabric 1 by a condensation reaction. The aqueous solution was removed after the reaction, and the resulting knitted fabric was washed with distilled water of 50°C and PBS (-) (manufactured by NISSUI PHARMACEUTICAL CO., LTD.).

The knitted fabric 1 was immersed in an aqueous solution containing 1.0 vol% of ethyl bromide and 30 vol% of methanol, and reacted at 35 to 50°C for 5 hours. Thus, the polyethyleneimine covalently bound to the surface of the knitted fabric 1 was converted to quaternary ammonium. After the polyethyleneimine was converted to quaternary ammonium, the aqueous solution was removed, and the resulting knitted fabric was washed with an aqueous solution containing 30 vol% methanol and distilled water.

An aqueous solution containing 54 international units/mL of dalteparin sodium (dalteparin Na intravenous injection 5000 units/5 mL "Sawai", manufactured by Sawai Pharmaceutical Co., Ltd.) and 0.1 mol/L sodium chloride was prepared, and the pH was adjusted to pH 4. The knitted fabric 1 was immersed in this aqueous solution and reacted at 70°C for 6 hours for ionic bond with polyethyleneimine. The aqueous solution was removed, the resulting knitted fabric was washed with distilled water, and then dried in vacuum. After the drying in vacuum, the resulting knitted fabric was sterilized with ethylene oxide gas. Thus, a medical base material 1 treated for antithrombotic property was obtained.

(Example 2)

A medical base material 2 was prepared by the same operation as in the method of Example 1 except that a flat knitted fabric having the number of wales of 50/2.54 cm and the number of courses of 50/2.54 cm after the removal treatment of the sea component was used instead of the flat knitted fabric having the number of wales of 70/2.54 cm and the number of courses of 70/2.54 cm after the removal treatment of the sea component.

(Example 3)

A medical base material 3 was prepared by the same operation as in the method of Example 1 except that a flat knitted fabric having the number of wales of 130/2.54 cm and the number of courses of 130/2.54 cm after the removal treatment of the sea component was used instead of the flat knitted fabric having the number of wales of 70/2.54 cm and the number of courses of 70/2.54 cm after the removal treatment of the sea component.

(Example 4)

A medical base material 4 was prepared by the same operation as in the method of Example 2 except that half tricot knitting was performed using a tricot knitting machine instead of flat knitting.

(Example 5)

A medical base material 5 was prepared by the same operation as in the method of Example 2 except that double denbigh knitting was performed using a tricot knitting machine instead of flat knitting.

(Example 6)

A medical base material 6 was prepared by the same operation as in the method of Example 2 except that atlas knitting was performed using a tricot knitting machine instead of flat knitting.

(Example 7)

A medical base material 7 was prepared by the same operation as in the method of Example 1 except that a multifilament yarn made of 18 filaments of sea-island composite fibers (6 islands/filament) with a total fineness of 132 dtex was used instead of a multifilament yarn made of 9 filaments of sea-island composite fibers (70 islands/filament) with a total fineness of 66 dtex.

(Example 8)

A multifilament yarn made of 18 filaments of sea-island composite fibers (6 islands/filament) with a total fineness of 132 dtex was used instead of a multifilament yarn made of 9 filaments of sea-island composite fibers (70 islands/filament) with a total fineness of 66 dtex. A medical base material 8 was prepared by the same operation as in the method of Example 1 except that a flat knitted fabric having the number of wales of 130/2.54 cm and the number of courses of 130/2.54 cm after the removal treatment of the sea component was used instead of the flat knitted fabric having the number of wales of 70/2.54 cm and the number of courses of 70/2.54 cm after the removal treatment of the sea component.

(Example 9)

A medical base material 9 was prepared by the same operation as in the method of Example 1 except that a multifilament yarn made of 9 filaments of sea-island composite fibers (70 islands/filament) with a total fineness of 66 dtex and a monofilament of 44 dtex was used instead of a multifilament yarn made of 9 filaments of sea-island composite fibers (70 islands/filament) with a total fineness of 66 dtex.

(Example 10)

A medical base material 10 was prepared by the same operation as in the method of Example 1 except that a multifilament yarn made of 9 filaments of sea-island composite fibers (70 islands/filament) with a total fineness of 66 dtex and a monofilament of 56 dtex was used instead of a multifilament yarn made of 9 filaments of sea-island composite fibers (70 islands/filament) with a total fineness of 66 dtex.

(Comparative Example 1)

A medical base material 11 was prepared by the same operation as in the method of Example 1 except that a flat knitted fabric having the number of wales of 50/2.54 cm and the number of courses of 50/2.54 cm after the removal treatment of the sea component was used instead of the flat knitted fabric having the number of wales of 70/2.54 cm and the number of courses of 70/2.54 cm after the removal treatment of the sea component.

(Comparative Example 2)

A medical base material 12 was prepared by the same operation as in the method of Example 1 except that a flat knitted fabric having the number of wales of 170/2.54 cm and the number of courses of 170/2.54 cm after the removal treatment of the sea component was used instead of the flat knitted fabric having the number of wales of 70/2.54 cm and the number of courses of 70/2.54 cm after the removal treatment of the sea component.

(Comparative Example 3)

A medical base material 13 was prepared by the same operation as in the method of Example 1 except that a multifilament yarn made of 18 filaments of sea-island composite fibers (6 islands/filament) with a total fineness of 132 dtex was used instead of a multifilament yarn made of 9 filaments of sea-island composite fibers (70 islands/filament) with a total fineness of 66 dtex.

(Comparative Example 4)

A multifilament yarn made of 18 filaments of sea-island composite fibers (6 islands/filament) with a total fineness of 132 dtex was used instead of a multifilament yarn made of 9 filaments of sea-island composite fibers (70 islands/filament) with a total fineness of 66 dtex. A medical base material 14 was prepared by the same operation as in the method of Example 1 except that a flat knitted fabric having the number of wales of 170/2.54 cm and the number of courses of 170/2.54 cm after the removal treatment of the sea component was used instead of the flat knitted fabric having the number of wales of 70/2.54 cm and the number of courses of 70/2.54 cm after the removal treatment of the sea component.

(Comparative Example 5)

A multifilament yarn made of 9 filaments of sea-island composite fibers (6 islands/filament) with a total fineness of 66 dtex was used instead of a multifilament yarn made of 9 filaments of sea-island composite fibers (70 islands/filament) with a total fineness of 66 dtex. A medical base material 15 was prepared by the same operation as in the method of Example 1 except that a flat knitted fabric having the number of wales of 130/2.54 cm and the number of courses of 130/2.54 cm after the removal treatment of the sea component was used instead of the flat knitted fabric having the number of wales of 70/2.54 cm and the number of courses of 70/2.54 cm after the removal treatment of the sea component.

(Comparative Example 6)

A multifilament yarn made of 6 filaments of sea-island composite fibers (6 islands/filament) with a total fineness of 132 dtex was used instead of a multifilament yarn made of 9 filaments of sea-island composite fibers (70 islands/filament) with a total fineness of 66 dtex. A medical base material 16 was prepared by the same operation as in the method of Example 1 except that a flat knitted fabric having the number of wales of 130/2.54 cm and the number of courses of 130/2.54 cm after the removal treatment of the sea component was used instead of the flat knitted fabric having the number of wales of 70/2.54 cm and the number of courses of 70/2.54 cm after the removal treatment of the sea component.

(Comparative Example 7)

A multifilament yarn made of 38 filaments with a total fineness of 66 dtex was used instead of a multifilament yarn made of 9 filaments of sea-island composite fibers (70 islands/filament) with a total fineness of 66 dtex. A medical base material 17 was prepared by the same operation as in the method of Example 1 except that a flat knitted fabric having the number of wales of 100/2.54 cm and the number of courses of 100/2.54 cm after the removal treatment of the sea component was used instead of the flat knitted fabric having the number of wales of 70/2.54 cm and the number of courses of 70/2.54 cm after the removal treatment of the sea component.

(Comparative Example 8)

The knitted fabric 1, which was a flat knitted fabric made of multifilaments containing ultra-fine fibers, was subjected to the same steps up to the "(c-2) alkali treatment step" described in Example 1, but was not subjected to an antithrombotic treatment. Thus, a medical base material 18 without the treatment for antithrombotic property was prepared.

(Experiment Examples)

The medical base materials 1 to 18 were measured for the items in the following (1) to (12). Among the obtained results, the measurement results of (1) to (6) are shown in Table 1, and the measurement results of (7) to (12) are shown in Table 2.

(1) Single thread diameter

Using a scanning electron microscope (manufactured by Hitachi High-Technologies Corporation), 10 multifilaments or 10 ultra-fine fibers in a multifilament were arbitrarily selected. At any one point of each fiber, the length of the portion where a line orthogonal to the long axis direction of the fiber overlaps with the fiber was defined as the single thread diameter, and the average value of the single thread diameters at 10 points was calculated. The average value was measured for each of the medical base materials 1 to 18 and used as the single thread diameter of each of the medical base materials 1 to 18.

(2) Thickness

According to JIS L1096 8.4 (2010), after the medical base materials 1 to 18 were left at a constant pressure of 0.7 kPa for 10 seconds, the measured values ($\mu$m) of the medical base materials 1 to 18 were read. For each, the value was measured at 5 points at random, and the arithmetic mean value was calculated. The value ($\mu$m) rounded off to the first decimal place was used as the thickness.

(3) Basis weight

The basis weight was measured according to JIS L1096 8.3.2 A method (2010). Two test pieces of 10 mm $\times$ 10 mm were collected from each of the medical base materials 1 to 18 and were measured for the weight in the standard state. From the weights of the two test pieces, the average value of the weight of the knitted fabric per 10 mm $\times$ 10 mm was calculated and used as the basis weight (mg/cm$^2$) of each of the medical base materials 1 to 18.

(4) Atomic analysis using XPS on the surface of medical base materials

The abundance ratio of sulfur atoms to the abundance of all the atoms on the surface of medical base materials 1 to 18 can be determined by XPS.

[Measurement conditions]

Equipment: ESCALAB220iXL (manufactured by VG Scientific)
Excitation X-rays: monochromatic AlK$\alpha$1, 2 lines (1486.6 eV)
X-ray diameter: 1 mm
The angle of escape of X electrons: 90° (inclination of the detector with respect to the surface of the medical base material)

The surface of the medical base material herein refers to, when the angle of escape of X electrons is under the measurement conditions of XPS, that is, the inclination of the detector with respect to the surface of the medical base material is 90°, the portion detected from the measured surface to the depth of 10 nm. Atomic information on the surface of the medical base material can be obtained from the binding energy value of the bound electrons in the substance, which is obtained by irradiating the surface of the medical base material with X-rays and measuring the energy of the generated photoelectrons, and information on the valence and binding state can be obtained from the energy shift of the peak of each binding energy value. Furthermore, the area ratio of each peak can be used for quantification, that is, the abundance ratio of each atom, valence, and binding state can be calculated.

Specifically, the S2p peak indicating the presence of sulfur atoms is found in the vicinity of the binding energy value of 161 eV to 170 eV. In the present invention, it has been found that the area ratio of the S2p peak to all the peaks is preferably 3.0 to 6.0 at%. When the surface of the medical base material was measured by X-ray electron spectroscopy (XPS), the abundance ratio of sulfur atoms with respect to the abundance of all the atoms was calculated by rounding off to the second decimal place.

(5) Number of wales and number of courses

According to JIS L1096 8.6 (2010), the medical base materials 1 to 18 were placed on a flat table. In each of the medical base materials 1 to 18, excluding unnatural wrinkles and tension, the number of wales and the number of courses in 5 different sites, 5 samples in total were counted, and the first decimal place was rounded off.

(6) Mesh opening

Square knitted fabric samples having a width of 1 cm and a length of 1 cm were cut out from the medical base materials 1 to 18. Thus, 18 knitted fabric samples were prepared. Double-sided tape was attached on the sample table of the scanning electron microscope. Then, each knitted fabric sample was attached onto the double-sided tape so that one side of the square knitted fabric samples and the double-sided tape would face each other. Using a scanning electron microscope TM3000 (manufactured by Hitachi High-Technologies Corporation), the sample table was leveled so that the incident electrons would hit the side of the knitted fabric sample perpendicularly. In this state, images were photographed at a magnification of 100 times. Thus, images of each of the medical base materials 1 to 18 taken from the direction perpendicular to the stitches of the knitted fabrics were obtained (see Figure 1). In the obtained images, 10 gaps in the stitches detected as a portion without any thread were randomly selected. Each of these 10 gaps was approximated by a circle to obtain 10 maximum circles inscribed in the gaps of the stitches. The diameters of the maximum circles inscribed in the 10 gaps were measured, and the average value of the diameters of the 10 maximum circles was calculated.

The method of circle approximation is not particularly limited as long as the circle is the maximum circle inscribed in a gap between the stitches of the knitted fabric. As an example, the method of circle approximation using the Max Inscribed Circles plug-in of the image analysis software ImageJ (manufactured by National Institutes of Health) is described here. More specifically, the photographed image is converted into an 8-bit image on ImageJ, and the contour of the fiber is emphasized by the Find Edges command. Then, the Threshold command and the Invert command are executed to obtain an image in which only the contour of the fiber is outlined in white. Next, the Max Inscribed Circles plug-in is executed on the image outlined in white, and the maximum circle inscribed in the gap can be obtained as an approximate circle.

(7) Measurement of the tensile elongation at break and the tensile elastic modulus

The measurement was performed according to JIS L 1096 8.14 A method (strip method) (2010). From medical base materials 1 to 18, 5 samples having a width of 1 cm and a length of 3 cm with the warp direction as the length direction were prepared and extended by a tensile testing machine of constant rate extension with a grip interval of 1 cm and at a tensile rate of 0.5 cm/min. The breaking force (N) and elongation at break (%) were measured. The results were plotted with the force on the vertical axis and the elongation at break on the horizontal axis. In the section where the value obtained by dividing the elongation at break (%) by 100 is 0.00 to 0.03 (rounded to the third digit of the decimal point), a straight line was approximated by the least squares method (Microsoft Excel), and the slope of the straight line was divided by the cross-sectional area calculated from the thickness (mm) and sample width (mm) of the medical base materials 1 to 18 to obtain the elastic modulus (Pa). This was performed on 5 samples, and the average values of the tensile elongation at break and tensile elastic modulus were calculated.

(8) Sheath storage property

The medical base materials 1 to 18 were cut into a disk shape having a diameter of 35 mm, and a circle having a radius of 0.9 mm was drawn concentrically with the center of the circle. Then, 18 metal wires with a diameter of 0.3 mm and a length of 10 cm were radially bonded at equal intervals of 20 degrees based on the center of the disk, with one end of each wire on the concentric circle. The medical base materials 1 to 18 were folded, and the 18 metal wires were bundled in parallel. The storage in a polyvinyl chloride tube having an outer diameter of 4.7 mm, an inner diameter of 4.35 mm, and a length of 50 mm was evaluated. When the assembly consisting of the medical base material and the metal wires could be completely stored in the tube, it was considered as pass, and when even a part of the assembly remained outside the tube, it was considered as fail.

(9) Water permeability at a pressure of 120 mmHg

The medical base materials 1 to 18 were cut to prepare 1 cm $\times$ 1 cm sample fragments. The sample fragments were sandwiched by 2 donut-shaped gaskets having a diameter of 3 cm with a diameter of 0.5 cm punched out so that liquid would not pass through except for the punched portion. This is stored in a housing for a circular filtration filter. Water filtered through reverse osmosis membrane having a temperature of 25°C is passed through this circular filtration filter for 2 minutes or more until the sample fragment is sufficiently hydrated. Under the conditions of a temperature of 25°C and a filtration differential pressure of 120 mmHg, the water filtered through reverse osmosis membrane was subjected to total external pressure filtration for 30 seconds, and the permeation amount (mL) of water permeating the portion with a diameter of 1 cm is measured. The permeation amount is calculated by rounding off the first decimal place. The permeation amount (mL) is converted into a value per unit time (min) and effective area ($cm^2$) of the sample fragment, and the water permeability at a pressure of 120 mmHg is measured. The two samples were thus measured and the average value was calculated.

(10) Antithrombotic property (concentration of thrombin-antithrombin complex (hereinafter, "TAT"))

The medical base materials 1 to 18 and the untreated knitted fabric 1 (positive subject) were punched into a disk shape having a diameter of 6 mm, washed with physiological saline at 37°C for 30 minutes, and then placed in a 2 mL microtube. Heparin sodium injection (manufactured by Ajinomoto Pharmaceuticals Co., Ltd.) was added to fresh human blood to a concentration of 0.4 IU/mL. Then, 2 mL of this human blood was added, followed by the incubation at 37°C for 2 hours. After the incubation, the medical base materials 1 to 18 were taken out and the concentration of TAT in blood was measured. When a coagulation thrombus is formed in the living body, the thrombus is dissolved by the action of a fibrinolytic reaction. The balance between the coagulation reaction and the fibrinolytic reaction determines whether or not the thrombus remains in the cardiovascular system. If the TAT is 1500 ng/mL or less, the thrombus is dissolved by the fibrinolytic reaction. Therefore, this was set as the upper limit that does not cause a problem even if a thrombus is formed, and TAT $\leq$ 1500 ng/mL was set as the pass criterion for antithrombotic property.

(11) Number of cell adhesions

The medical base materials 1 to 18 were punched into a disk sample having a diameter of 15 mm with a punch. One piece was placed in a well of a 24-well microplate (manufactured by Sumitomo Bakelite Co., Ltd.) for cell culture with the inner wall surface facing up, and a metal pipe-shaped weight having a wall thickness of 1 mm was placed above. Normal human umbilical vein endothelial cells (manufactured by Takara Bio Inc.) suspended in endothelial cell medium kit-2 (2% FBS) (EGM TM-2 Bullet Kit (registered trademark); manufactured by Takara Bio Inc.) were added in an amount of $1 \times 10^4$ cells per well. The cells were cultured in 1 mL of the medium at 37°C in an environment of 5% $CO_2$ for 24 hours. Then, after rinsing with PBS (-) (manufactured by NISSUI PHARMACEUTICAL CO.,LTD.), 100 $\mu$L of Cell Counting Kit-8 (manufactured by DOJINDO LABORATORIES) was added, and then cultured at the temperature of 37°C for 4 hours in an environment of 5% $CO_2$. After the cell culture, the medical base materials 1 to 18 were measured at an absorbance of 450 nm by a microplate reader (MTP-300; manufactured by Corona Electric Co., Ltd.), and the absorbance was calculated as shown in the following formula 2.

$$As = At\text{-}Ab \; ... \quad Formula\ 2$$

At: Absorbance of measured value
Ab: Absorbance of blank solution (medium and Cell Counting Kit-8 solution only, no cells)
As: Calculated absorbance

The amount of the cell growth (cell/cm$^2$) after the cell culture can be calculated from the calculated absorbance As, using a calibration curve obtained by measuring the absorbance of known numbers of cells. Thus, the number of cell adhesions (cell/cm$^2$) on the medical base materials 1 to 18 was determined based on the absorbance As. Communication between cells is important for the proliferation of vascular endothelial cells. It has been reported that a cell density of 10$^3$ cells/cm$^2$ or more is required to promote proliferation on the surface of a polyester material or the like. In conventional indwelling cardiovascular devices, the inability of endothelial cells to adhere in 24 hours is one of the causes of inadequate endothelialization. The lack of the progress of endothelialization has caused clinical problems such as cerebral infarction and vascular occlusion. In order to form an endothelium on the surface of an indwelling cardiovascular device, it is necessary for adherent cells to proliferate by communication between cells. As the minimum number of adherent cells required for proliferation, 10$^3$ cells/cm$^2$ or more was used as the criterion for judging "adhesive."

(12) Endothelialization rate

The medical base materials 1 to 18 were rolled to a tube having an outer diameter of 3.3 mm and a length of 3 cm, and the outer circumference of the tube was sealed with Teflon (registered trademark) sealing tape to create an artificial blood vessel. A beagle dog was anesthetized by inhalation of isoflurane, and 100 IU/kg of heparin was intravenously administered. The artificial blood vessel was transplanted into the carotid artery by end-to-end anastomosis. Then, 30 days after the transplantation, the animal were euthanized by exsanguination under isoflurane inhalation anesthesia, and then the artificial blood vessel was removed. The artificial blood vessel was cut out in the length direction and fixed with 10% neutral buffered formalin. A paraffin-embedded section was prepared by a conventional method and stained with hematoxylin and eosin to prepare a tissue specimen. The Tissue specimen was imaged under a light microscope. The total length of the artificial blood vessel and the length to the tip of the endothelial cells coating the inner surface were measured, and the endothelialization rate was calculated as shown in the following formula 3. In conventional indwelling cardiovascular devices, the endothelialization progresses only by about 20 to 30%, which has caused clinical problems such as cerebral infarction and vascular occlusion. A higher rate of endothelialization results in a lower risk of complications since the origin of thrombus formation is reduced. The endothelialization of 75% or more can reduce the incidence of infarction or occlusion by 50% or more. Thus, the endothelialization rate of 75% or more, which can sufficiently prevent the thrombus formation, was used as a criterion for judging that "endothelialization was promoted".

$$E = Le \, / \, Lt \times 100 \, \dots \quad \text{Formula 3}$$

E: Endothelialization rate (%)
Le: Length to the tip of endothelial cells coating the inner surface of the tube (cm)
Lt: Overall length of the tube (cm)

[Table 1]

| Knitted fabric No. | Sea-island composite fibers | Monofilament F: Filament | Knitting | Ultra-fine fiber ratio | (1) Single thread diameter | (2) Thickness | (3) Basis weight | (4) Abundance ratio of sulfur atoms to abundance of all the atoms on medical base material surface | (5) Number of courses | (5) Number of wales | (6) Mesh opening |
|---|---|---|---|---|---|---|---|---|---|---|---|
| | T: dtex F: Filament | | | wt% | μm | μm | mg/cm$^2$ | at% | /inch | /inch | μm |
| | (): islands/ filament | | | ≥30% | 1 to 10 | ≤200 | 5-20 | 3-6 | 50-130 | 50-130 | ≤80 |

| | Knitted fabric No. | Sea-island composite fibers | Monofilament F: Filament | Knitting | Ultra-fine fiber ratio | (1) Single thread diameter | (2) Thickness | (3) Basis weight | (4) Abundance ratio of sulfur atoms to abundance of all the atoms on medical base material surface | (5) Number of courses | (5) Number of wales | (6) Mesh opening |
|---|---|---|---|---|---|---|---|---|---|---|---|---|
| | | T: dtex F: Filament | | | wt% | μm | μm | mg/cm² | at% | /inch | /inch | μm |
| | | (): islands/ filament | | | ≥30% | 1 to 10 | ≤200 | 5-20 | 3-6 | 50-130 | 50-130 | ≤80 |
| Examples | 1 | 66T-9F (70) | - | Flat knitting | 100 | 3 | 149 | 5.5 | 3.3 | 70 | 70 | 56 |
| | 2 | 66T-9F (70) | - | Flat knitting | 100 | 3 | 115 | 5 | 3.1 | 50 | 50 | 78 |
| | 3 | 66T-9F (70) | - | Flat knitting | 100 | 3 | 192 | 13.1 | 4.4 | 130 | 130 | 44 |
| | 4 | 66T-9F (70) | - | Half tricot knitting | 100 | 3 | 181 | 16.4 | 4.6 | 130 | 130 | 39 |
| | 5 | 66T-9F (70) | - | Double denbigh knitting | 100 | 3 | 195 | 18.5 | 4.2 | 130 | 130 | 37 |
| | 6 | 66T-9F (70) | - | Atlas knitting | 100 | 3 | 192 | 17.3 | 4.5 | 130 | 130 | 33 |
| | 7 | 132T-18F (6) | - | Flat knitting | 100 | 10 | 152 | 9.2 | 5.5 | 70 | 70 | 64 |
| | 8 | 132T-18F (6) | - | Flat knitting | 100 | 10 | 188 | 19.8 | 5.5 | 130 | 130 | 24 |
| | 9 | 66T-9F (70) | 44T | Flat knitting | 80 | 3 | 159 | 6.8 | 3.5 | 70 | 70 | 52 |
| | 10 | 66T-9F (70) | 56T | Flat knitting | 40 | 3 | 161 | 7.2 | 3.5 | 70 | 70 | 49 |

| | Knitted fabric No. | Sea-island composite fibers | Monofilament F: Filament | Knitting | Ultra-fine fiber ratio | (1) Single thread diameter | (2) Thickness | (3) Basis weight | (4) Abundance ratio of sulfur atoms to abundance of all the atoms on medical base material surface | (5) Number of courses | (5) Number of wales | (6) Mesh opening |
|---|---|---|---|---|---|---|---|---|---|---|---|---|
| | | T: dtex F: Filament | | | wt% | μm | μm | mg/cm² | at% | /inch | /inch | μm |
| | | (): islands/filament | | | ≥30% | 1 to 10 | ≤200 | 5-20 | 3-6 | 50-130 | 50-130 | ≤80 |
| Comparative Examples | 1 | 66T-9F (70) | - | Flat knitting | 100 | 3 | 110 | 3.8 | 3.3 | 50 | 50 | 95 |
| | 2 | 66T-9F (70) | - | Flat knitting | 100 | 3 | 249 | 21.1 | 4.4 | 170 | 170 | 20 |
| | 3 | 132T-18F (6) | - | Flat knitting | 100 | 10 | 122 | 4.8 | 3.9 | 50 | 50 | 85 |
| | 4 | 132T-18F (6) | - | Flat knitting | 100 | 10 | 263 | 27.2 | 4.5 | 170 | 170 | 15 |
| | 5 | 66T-9F (6) | - | Flat knitting | 100 | 10 | 180 | 4.1 | 3.9 | 130 | 130 | 71 |
| | 6 | 132T-6F (6) | - | Flat knitting | 100 | 9 | 176 | 4.7 | 3.7 | 130 | 130 | 66 |
| | 7 | 66T-38F | - | Flat knitting | 0 | 15 | 215 | 23 | 4.6 | 100 | 100 | 76 |
| | 8 | 66T-9F (70) | - | Flat knitting | 100 | 3 | 148 | 5.7 | 0.0 | 70 | 70 | 58 |

EP 3 919 664 A1

[Table 2]

| Knitted fabric No. | Sea-island composite fibers T: dtex F: Filament (): islands/filament | Monofilament F: Filament | Knitting | Ultra-fine fiber ratio wt% ≥30% | (7) Tensile elongation at break % ≤20 | (7) Tensile elastic modulus MPa 1-100 | (8) Sheath storage property | (9) Water permeability at a pressure of 120 mmHg mL/min/cm² 1000-10000 | (10) Antithrombotic property (TAT) concentration ng/mL/cm² ≤1500 | (11) Number of cell adhesions cell/cm² >1000 | (12) Endothelization rate % ≥75 |
|---|---|---|---|---|---|---|---|---|---|---|---|
| 1 | 66T-9F (70) | - | Flat knitting | 100 | 17 | 45 | Pass | 8710 | 990 | 3190 | 83 |
| 2 | 66T-9F (70) | - | Flat knitting | 100 | 18 | 21 | Pass | 9560 | 1410 | 3050 | 81 |
| 3 | 66T-9F (70) | - | Flat knitting | 100 | 15 | 69 | Pass | 7830 | 710 | 3540 | 80 |
| 4 | 66T-9F (70) | - | Half tricot knitting | 100 | 11 | 72 | Pass | 7590 | 670 | 3750 | 88 |
| 5 | 66T-9F (70) | - | Double denbigh knitting | 100 | 8 | 85 | Pass | 7220 | 650 | 4510 | 93 |
| 6 | 66T-9F (70) | - | Atlas knitting | 100 | 9 | 81 | Pass | 7300 | 720 | 4440 | 92 |
| 7 | 132T-18F (6) | - | Flat knitting | 100 | 11 | 76 | Pass | 8390 | 910 | 4870 | 94 |
| 8 | 132T-18F (6) | - | Flat knitting | 100 | 5 | 95 | Pass | 7060 | 610 | 4870 | 94 |
| 9 | 66T-9F (70) | 44T | Flat knitting | 80 | 15 | 96 | Pass | 8540 | 920 | 3200 | 84 |
| 10 | 66T-9F (70) | 56T | Flat knitting | 40 | 14 | 98 | Pass | 3400 | 890 | 3150 | 83 |

Examples

16

| | Knitted fabric No. | Sea-island composite fibers<br><br>T: dtex F: Filament<br><br>(): islands/ filament | Monofilament F: Filament | Knitting | Ultra-fine fiber ratio<br><br>wt%<br><br>≥30% | (7) Tensile elongation at break<br><br>%<br><br>≤20 | (7) Tensile elastic modulus<br><br>MPa<br><br>1-100 | (8) Sheath storage property | (9) Water permeability at a pressure of 120 mmHg<br><br>mL/min/cm²<br><br>1000-10000 | (10) Antithrombotic property (TAT) concentration<br><br>ng/mL/cm²<br><br>≤1500 | (11) Number of cell adhesions<br><br>cell/cm²<br><br>>1000 | (12) Endothelization rate<br><br>%<br><br>≥75 |
|---|---|---|---|---|---|---|---|---|---|---|---|---|
| Comparative Examples | 1 | 66T-9F (70) | - | Flat knitting | 100 | 40 | 21 | Pass | 11200 | 1890 | 660 | 31 |
| | 2 | 66T-9F (70) | - | Flat knitting | 100 | 3 | 119 | Fail | 980 | 650 | 5820 | 87 |
| | 3 | 132T-18F (6) | - | Flat knitting | 100 | 35 | 42 | Pass | 10300 | 1520 | 990 | 41 |
| | 4 | 132T-18F (6) | - | Flat knitting | 100 | 1 | 145 | Fail | 870 | 620 | 5710 | 99 |
| | 5 | 66T-9F (6) | - | Flat knitting | 100 | 36 | 29 | Pass | 7790 | 730 | 770 | 90 |
| | 6 | 132T-18F (6) | - | Flat knitting | 100 | 30 | 33 | Pass | 7610 | 740 | 830 | 37 |
| | 7 | 66T-38F | - | Flat knitting | 0 | 28 | 105 | Fail | 7640 | 840 | 870 | 72 |
| | 8 | 66T-9F (70) | - | Flat knitting | 100 | 17 | 46 | Pass | 8610 | 5700 | 3530 | 25 |

As shown in Tables 1 and 2, by controlling the knitting density of a knitted fabric made of multifilaments containing ultra-fine fibers and immobilizing heparin or the like on the surface of the ultra-fine fibers, a medical base material having storage property in a sheath catheter and mechanical strength as well as antithrombotic property due to early endothelialization, which were not possible to achieve at the same time in the conventional technique, can be provided.

Industrial Applicability

The medical base material for an indwelling cardiovascular device of the present invention can be suitably used for an indwelling device for an endovascular treatment, and in particular, can be used for a medical device for cardiovascular implants such as a left atrial appendage occlusion device, an artificial valve and a stent graft.

**Claims**

1. A medical base material for an indwelling cardiovascular device, comprising a knitted fabric made of multifilaments containing 30 wt% or more of ultra-fine fibers having a single thread diameter of 1 $\mu$m to 10 $\mu$m, and heparin, a heparin derivative, or a pharmaceutically acceptable salt thereof, which is chemically bound to the surface of said ultra-fine fibers, wherein:

   said knitted fabric has a basis weight of 5 mg/cm$^2$ to 20 mg/cm$^2$, a thickness of 200 $\mu$m or less; and
   a water permeability of 1000 mL/min/cm$^2$ to 10,000 mL/min/cm$^2$ at a pressure of 120 mmHg.

2. The medical base material according to claim 1, wherein said knitted fabric has 50 to 130 courses per 2.54 cm and 50 to 130 wales per 2.54 cm.

3. The medical base material according to claim 1 or 2, wherein the average diameter of maximum circles inscribed in gaps between stitches of said knitted fabric is 80 $\mu$m or less.

4. The medical base material according to any one of claims 1 to 3, wherein said knitted fabric has a tensile elongation at break of 50% or more.

5. The medical base material according to any one of claims 1 to 4, wherein said knitted fabric has a tensile elastic modulus of 1 MPa to 100 MPa.

Fig. 1

| | | International application No. |
|---|---|---|
| **INTERNATIONAL SEARCH REPORT** | | PCT/JP2020/003358 |

**A. CLASSIFICATION OF SUBJECT MATTER**

D04B 1/16(2006.01)i; D04B 21/16(2006.01)i; A61F 2/01(2006.01)i; A61F 2/04(2013.01)i; A61L 33/10(2006.01)i; A61B 17/12(2006.01)i; D06M 15/03(2006.01)i
FI:    A61L33/10; A61B17/12; A61F2/01; A61F2/04; D04B1/16; D04B21/16; D06M15/03

According to International Patent Classification (IPC) or to both national classification and IPC

**B. FIELDS SEARCHED**

Minimum documentation searched (classification system followed by classification symbols)
D04B1/16; D04B21/16; A61F2/01; A61F2/04; D06M15/03; A61L33/10; A61B17/12

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | 野一色泰晴ほか，超極細繊維交絡型人工血管の開発，人工臓器，15 February 1999, vol. 28, no. 1, pp. 278-283, ISSN:0037-2188, summary, page 279, left column, lines 27-42, page 279, right column, lines 2-3 from the bottom, page 281, right column, lines 2-3, fig. 1, (NOISHIKI, Yasuharu et al., "DEVELOPMENT OF A KNITTED FABRIC VASCULARPROSTHESIS IMPREGNATED WITH ULTRAFINE POLYES TER FIBERS", Jinko Zoki) | 1-5 |
| Y | JP 2011-522615 A (NELLIX, INC.) 04.08.2011 (2011-08-04) paragraph [0068] | 1-5 |

☒ Further documents are listed in the continuation of Box C.　　☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 23 March 2020 (23.03.2020) | 07 April 2020 (07.04.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2020/003358

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 62-258670 A (UBE INDUSTRIES, LTD.) 11.11.1987 (1987-11-11) page 4, lower left column, lines 4-10 | 1-5 |
| Y | JP 61-263448 A (HIROYOSHI, Toshiki) 21.11.1986 (1986-11-21) page 3, upper right column, lines 13-20 | 1-5 |
| Y | JP 2006-508773 A (CARDIO INCORPORATED) 16.03.2006 (2006-03-16) paragraph [0162] | 1-5 |
| Y | 井上真理，繊維集合体としての布の特性について，繊維学会誌，　30 September 2008, vol. 64, no. 8, pp. 246-251, ISSN:0037-9875, page 247, left column, lines 8-18, (INOUE, Mari, "Research on the Mechanics of Fibrous Materials", Sen'i Gakkaishi) | 1-5 |

Form PCT/ISA/210 (continuation of second sheet) (January 2015)

**INTERNATIONAL SEARCH REPORT**
Information on patent family members

International application No.

PCT/JP2020/003358

| Patent Documents referred in the Report | Publication Date | Patent Family | Publication Date |
|---|---|---|---|
| JP 2011-522615 A | 04 Aug. 2011 | US 2009/0318949 A1 paragraph [0090] CA 2726596 A paragraph [0086] WO 2009/149294 A1 paragraph [0086] | |
| JP 62-258670 A | 11 Nov. 1987 | (Family: none) | |
| JP 61-263448 A | 21 Nov. 1986 | (Family: none) | |
| JP 2006-508773 A | 16 Mar. 2006 | US 2006/0252981 A1 paragraph [0204] WO 2004/050133 A2 page 51, line 10 to page 52, line 8 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016068279 A
- WO 2015080177 A
- WO 2014168198 A
- JP 2001254250 A
- JP 2000265343 A
- WO 2013065688 A
- JP 2017206790 A
- JP 2018172813 A
- US 3531368 A

- US 3350488 A
- JP 4152075 B
- JP 3497612 B
- JP H10513074 A
- JP S60041947 B
- JP S60047287 B
- JP 4273965 B
- JP H10151192 A

**Non-patent literature cited in the description**

- **MAREK GRYGIER et al.** *Advances in Interventional Cardiology,* 2017, vol. 13 (42), 62-66